(19) Europäisches Patentamt
European Patent Office
Office européen des brevets

(11) **EP 3 202 420 B1**

(12) **EUROPEAN PATENT SPECIFICATION**

(45) Date of publication and mention
of the grant of the patent:
**04.03.2020 Bulletin 2020/10**

(51) Int Cl.:
*A61K 9/06* (2006.01)   *A61K 47/10* (2017.01)
*A61K 47/12* (2006.01)   *A61K 31/138* (2006.01)
*A61P 35/00* (2006.01)

(21) Application number: **17157911.3**

(22) Date of filing: **09.12.2009**

(54) **TRANSDERMAL PHARMACEUTICAL COMPOSITIONS COMPRISING A SERM**

TRANSDERMALE PHARMAZEUTISCHE ZUSAMMENSETZUNGEN MIT EINEM SERM

COMPOSITIONS PHARMACEUTIQUES TRANSDERMIQUES COMPORTANT UN STROGÈNE SERM

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.12.2008 EP 08305924**
**11.12.2008 US 121848 P**

(43) Date of publication of application:
**09.08.2017 Bulletin 2017/32**

(60) Divisional application:
**20151652.3**

(62) Document number(s) of the earlier application(s) in accordance with Art. 76 EPC:
**09764869.5 / 2 373 305**

(73) Proprietor: **Besins Healthcare Luxembourg SARL 2668 Luxembourg (LU)**

(72) Inventor: **Masini-Eteve, Valérie**
**92340 Bourg La Reine (FR)**

(74) Representative: **Nederlandsch Octrooibureau**
**P.O. Box 29720**
**2502 LS The Hague (NL)**

(56) References cited:
**WO-A-2008/070463    WO-A1-99/24041**

US-A1- 2004 175 416

- YOUNG CHAI LIM ET AL: "Endoxifen (4-hydroxy-N-desmethyl-tamoxifen) has anti-estrogenic effects in breast cancer cells with potency similar to 4-hydroxy-tamoxifen", CANCER CHEMOTHERAPY AND PHARMACOLOGY, SPRINGER, BERLIN, DE, vol. 55, no. 5, 1 May 2005 (2005-05-01), pages 471-478, XP019334143, ISSN: 1432-0843
- LEE K-H ET AL: "Quantification of tamoxifen and three metabolites in plasma by high-performance liquid chromatography with fluorescence detection: application to a clinical trial", JOURNAL OF CHROMATOGRAPHY B: BIOMEDICAL SCIENCES & APPLICATIONS, ELSEVIER, AMSTERDAM, NL, vol. 791, no. 1-2, 5 July 2003 (2003-07-05), pages 245-253, XP004428049, ISSN: 1570-0232
- MICHAEL D JOHNSON ET AL: "Pharmacological Characterization of 4-hydroxy-N-desmethyl Tamoxifen, a Novel Active Metabolite of Tamoxifen", BREAST CANCER RESEARCH AND TREATMENT, KLUWER ACADEMIC PUBLISHERS, BO, vol. 85, no. 2, 1 May 2004 (2004-05-01), pages 151-159, XP019274612, ISSN: 1573-7217

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to transdermal SERM-containing pharmaceutical compositions, such as gels, and to methods of making and using the same.

**BACKGROUND**

The present invention relates to SERM-containing pharmaceutical compositions, such as gels, and to methods of making and using the same.

The Selective Estrogen Receptors Modulators (SERMs) are a class of pharmacological agents formerly referred to as anti-estrogens, which are generally understood to be compounds capable of blocking the effect of estradiol without displaying any estrogenic activity of their own. Such a description is now known to be incomplete, however. The term SERM has been coined to describe compounds that, in contrast to pure estrogen agonists or antagonists, have a mixed and selective pattern of estrogen agonist-antagonist activity, which largely depends on the targeted tissue. The pharmacological goal of these drugs is to produce estrogenic actions in those tissues where these actions are beneficial (such as bone, brain, liver) and to have either no activity or antagonistic activity in tissues such as breast and endometrium, where estrogenic actions (cellular proliferation) might be deleterious.

SERMs have been commercialized for oral administration. However, the oral administration of Active Principles such as SERMs leads to gastrointestinal side effects and degradation of the active substance by liver enzymes. SERMs are significantly metabolized upon first liver passage and some of the obtained metabolites are not as active as the SERM itself. Thus, some amount of orally administered SERMs may be metabolized into inactive products before reaching their final target.

Therefore, the transdermal administration of medicinal active principles represents a persuasive technique since it is non- invasive and is endowed with certain advantages such as the avoidance of the poor gastrointestinal absorption, the high hepatic extraction and high first pass effect.

Another advantage of transdermal application is to ensure that active agents selectively reach the corresponding target tissue or areas to be treated as quickly as possible. It can be particularly interesting in the case of local estrogen imbalance such as localized tumours, such as breast tumours for example. In these cases, administration of SERMs requires specific targeting to tissues.

WO 2008/070463 A discloses hydroalcoholic compositions for topical administration comprising endoxifen (0.01-0.20% w/w), the penetration enhancer isopropyl myristate (0.1-2.0% w/w), an alcohol (ethanol or isopropanol) (50.0-80.0% w/ w), an aqueous vehicle (phosphate buffered solution) (20.0-60.0% w/w) and a gelling agent (selected from polyacrylic acid, hydroxypropylcellulose and a cellulose derivative other than hydroxypropylcellulose) (1.0-10.0% w/w). The document also discloses the use of said compositions for the treatment and inhibition of breast diseases, in particular breast cancer.

The present invention provides compositions suitable for non-oral administration of SERMs, and related methods of making and using them.

**SUMMARY**

The present invention provides a transdermal pharmaceutical composition for transdermal administration to a skin surface wherein said composition comprises: (i) 0.01 to 10 % (w/w), preferably 2 to 5 % (w/w) of endoxifen or a pharmaceutically acceptable salt thereof, (ii) 60 to 80 % (w/w) of at least one monoalcohol, wherein the at least one monoalcohol is selected from C2-C4 monoalcohols, preferably, ethanol or isopropanol, (iii) 0.01 to 10 % (w/w) of at least one penetration enhancer, wherein the at least one penetration enhancer is oleic acid and with the proviso of isopropyl myristate being excluded, (iv) 0 to 5 % (w/w) of at least one gelling agent, preferably, polyacrylic acids, cellulosics, or mixtures thereof, and (v) q.s. 100 % (w/w) aqueous vehicle.

The monoalcohol may be selected from the group consisting of ethanol and isopropanol.

The penetration enhancer is oleic acid with the proviso of isopropyl myristate being excluded. In another embodiment, the penetration enhancer is a mixture oleic acid and propylene glycol, with the proviso of isopropyl myristate being excluded.

The gelling agent may be selected from the group consisting of polyacrylic acids, cellulosics, and mixtures thereof.

The invention also provides a pharmaceutical composition as described above and elsewhere herein for treating a patient in need thereof, such as a patient suffering from estrogen imbalance or a breast disorder, or a patient suffering from benign breast diseases, gynecomastia, breast cancer, mastalgia and conditions involving dense breast tissue.

The invention also provides the use of a SERM selected from the group consisting of endoxifen and the pharmaceutically acceptable salts thereof, in the preparation of a pharmaceutical composition as described above and elsewhere herein, wherein the pharmaceutical composition is for treating a patient suffering from a breast disorder selected from benign breast diseases, gynecomastia, breast cancer, mastalgia and conditions involving dense breast tissue.

The invention also provides a dose packet, unit dose packet or multiple dose packet containing a pharmaceutical composition as described above and elsewhere herein.

The invention also provides a dispenser, optionally with hand pump, containing a pharmaceutical composition as described above and elsewhere herein.

In one embodiment, there is a provided a process for preparing a pharmaceutical composition as described above and elsewhere herein comprising the steps of preparing a mixture comprising:
(i) at least one monoalcohol selected from C2-C4 monoalcohols, (ii) endoxifen or the pharmaceutically acceptable salts thereof, (iii) an aqueous vehicle, (iv) at least one penetration enhancer, wherein the at least one penetration enhancer is oleic acid and with the proviso of isopropyl myristate being excluded, and (v) optionally, at least one gelling agent.

The invention provides a transdermal pharmaceutical composition as described above and elsewhere herein, for use in a method of administering a therapeutically effective amount of a SERM selected from the group consisting of endoxifen and the pharmaceutically acceptable salts thereof, to a patient in need thereof, comprising transdermally administering to a surface of skin of the patient the pharmaceutical composition as described above and elsewhere herein.

## DETAILED DESCRIPTION

For the purposes of this disclosure and unless otherwise specified, "a" or "an" means "one or more."

Unless otherwise stated, percentages (%) refer to amounts by weight based upon total weight of the composition (w/w).

In accordance with one aspect, the invention provides a pharmaceutical composition comprising a SERM selected from the group consisting of endoxifen and the pharmaceutically acceptable salts thereof. The composition is suitable for transdermal or transcutaneous delivery.

Formulating drugs for percutaneous delivery is a difficult and unpredictable art. In particular, the formulation of hormones, steroids, and steroid-like compounds for percutaneous delivery has proven delicate as each active ingredient tends to behave differently in terms of transdermal flux, for example.

The compositions described herein were surprisingly and unexpectedly found to yield good results in *in vitro* experiments measuring transdermal flux of the SERM in Franz cells, as illustrated in the examples. As described in more detail below, *in silico* predictions for transdermal flux of the SERM were carried out but could not predict *in vitro* experimental results.

The transdermal pharmaceutical composition of the invention for transdermal administration to a skin surface comprises: (i) 0.01 to 10 % (w/w), preferably 2 to 5 % (w/w) of endoxifen or a pharmaceutically acceptable salt thereof, (ii) 60 to 80 % (w/w) of at least one monoalcohol, wherein the at least one monoalcohol is selected from C2-C4 monoalcohols, preferably, ethanol or isopropanol, (iii) 0.01 to 10 % (w/w) of at least one penetration enhancer, wherein the at least one penetration enhancer is oleic acid and with the proviso of isopropyl myristate being excluded, (iv) 0 to 5 % (w/w) of at least one gelling agent, preferably, polyacrylic acids, cellulosics, or mixtures thereof, and (v) q.s. 100 % (w/w) aqueous vehicle.

In some embodiments, the compositions according to the invention do not require any adhesive, matrix or membrane for administration, by contrast to patches or occlusive systems. Such embodiments offer clear advantages over known compositions that require an adhesive, such as avoiding the use of potentially irritating ingredients.

In some embodiments, the compositions of the invention offer further advantages, including being non-irritating to the skin and resulting in limited side effects. The compositions of the invention may allow a local, non systemic delivery of the SERM and result in a local effect of the drug which avoids systemic side effects, such as gastrointestinal irritation or effects attributable to androgenic activity, such as acne, oily skin or hair, hirsutism, weight gain, deepening of the voice, and androgenic alopecia.

As a result of these and other advantages, the compositions facilitate patient compliance.

### Compositions

As noted above, the transdermal pharmaceutical composition for transdermal administration to a skin surface of the present invention comprises: (i) 0.01 to 10 % (w/w), preferably 2 to 5 % (w/w) of endoxifen or a pharmaceutically acceptable salt thereof, (ii) 60 to 80 % (w/w) of at least one monoalcohol, wherein the at least one monoalcohol is selected from C2-C4 monoalcohols, preferably, ethanol or isopropanol, (iii) 0.01 to 10 % (w/w) of at least one penetration enhancer, wherein the at least one penetration enhancer is oleic acid and with the proviso of isopropyl myristate being excluded, (iv) 0 to 5 % (w/w) of at least one gelling agent, preferably, polyacrylic acids, cellulosics, or mixtures thereof, and (v) q.s. 100 % (w/w) aqueous vehicle.

In some embodiments, the composition comprises the specified components. In other embodiments, the composition consists of the specified components. In yet other embodiments, the composition consists essentially of the specified components. As used herein, "consists essentially of" the specified components means that the composition includes at least the specified components, and may also include other components that do not materially affect the basic and novel characteristics of the components.

As noted above, compositions of the invention are suitable for transdermal administration. For example, the compositions can be directly applied to a surface of the skin, for direct non-occlusive transdermal/transcutaneous application. As used herein, the terms "direct"/"directly" and "non-occlusive" reflect that the compositions of the invention do not require a matrix or membrane to effect administration, and thus are not required to be dispensed via a patch, plaster, tape system, or the like. However, the compositions of the invention can be dispensed via a patch, plaster, tape system or the like.

In some embodiments, the amount of composition administered is a defined, finite amount that provides a therapeutically effective amount (such as a single daily dose) of the SERM.

As used herein, the phrase "therapeutically effective amount" means an amount (dosage) that achieves the specific pharmacological response for which the drug is administered in a given patient. It is emphasized that a "therapeutically effective amount" of a drug that is administered to a particular subject in a particular instance may not always be effective in treating the target conditions/diseases, even though such dosage is deemed to be a therapeutically effective amount by those of skill in the art. Those skilled in the art will recognize that the "therapeutically effective amount" may vary from patient to patient, or from condition to condition, and can determine a "therapeutically effective amount" for a given patient/condition by routine means.

In some embodiments, the composition is administered to a surface of the skin over a defined surface area. The administration of a defined, finite amount of the composition to a defined surface area permits the control of the amount of active principle, i.e., the SERM, that is applied to a given surface area, thereby controlling the local concentration. By controlling (e.g., limiting) local concentration, local side effects, such as local androgenic effects (including but not limited to acne and oily skin), can be minimized.

**SERM**

As noted above, the compositions of the invention comprise a therapeutically effective amount of a SERM selected from the group consisting of endoxifen and the pharmaceutically acceptable salts thereof.

The composition of the invention comprises 0.01 to 10 % (w/w) of the SERM, for example 0.05 to 5% (w/w), and for example 0.5 to 1.5 % (w/w) of the SERM. Typically, the composition of the invention may comprise 0.05 %, 0.1 %, 0.2 %, 0.3 %, 0.4 %, 0.5 %, 0.6 %, 0.7 %, 0.8 %, 0.9 %, 1 %, 1.5 %, or 2% (w/w) of the SERM.

As used herein, the term "endoxifen" refers to 4-hydroxy-N-desmethyl-tamoxifen and constitutes a secondary metabolite of tamoxifen. The composition of the invention comprises 0.01 to 10 % (w/w) of endoxifen, for example 0.5 to 6% (w/w), and for example 2 to 5 % (w/w) of endoxifen.

Those skilled in the art will understand that pharmaceutically acceptable active enantiomers, isomers, tautomers, salts, chelates, and amides of the SERM also can be used.

**Monoalcohols**

As noted above, the compositions of the invention comprise at least one monoalcohol, selected from C2-C4 monoalcohols. As used herein the term "monoalcohol" refers to an organic molecule containing at least one carbon atom and one only alcohol group -OH.

Exemplary C2-C4 monoalcohols are ethanol, n-propanol, isopropanol, n-butanol, isobutanol, tert-butanol, or mixtures thereof. In a preferred embodiment, monoalcohols suitable for the compositions of the invention are ethanol and isopropanol.

The presence of such a monoalcohol can act as a solvent for the SERM. Typically, the greater the amount of the SERM in the composition, the more monoalcohol will be used to solubilize the SERM.

The presence of such a monoalcohol may also contribute to accelerate drying of the composition onto the skin.

The monoalcohol will be used in an amount between 60 to 80 % (w/w) of the total composition, for example in an amount of 60%, 65%, 70%, 75% or 80% of the total composition.

**Penetration enhancer**

A "penetration enhancer" is an agent known to accelerate the delivery of the drug through the skin. These agents can also be referred to as accelerants, adjuvants, and absorption promoters, and are collectively referred to herein as "enhancers". This class of agents includes those with diverse mechanisms of actions including those which have the function of improving the solubility and diffusibility of the drug, and those which improve percutaneous absorption by changing the ability of the stratum corneum to retain moisture, softening the skin, improving the skin's permeability, acting as penetration assistants or hair-follicle openers or changing the state of the skin, such as changing the state or fluidity of the stratum corneum intercellular lipid bilayers.

Penetration enhancers may be functional derivatives of a fatty acid, which includes isosteric modifications of fatty acids or non-acidic derivatives of the carboxylic functional group of a fatty acid or isosteric modifications thereof. In one embodiment, the functional derivative of a fatty acid is an unsaturated alkanoic acid in which the -COOH group is substituted with a functional derivative thereof, such as alcohols, polyols, amides and substituted derivatives thereof. The term "fatty acid" means a fatty acid that has four (4) to twenty-four (24) carbon atoms. Non-limiting examples of penetration enhancers include C8-C22 fatty acids such as isostearic acid, octanoic acid, and oleic acid; C8-C22 fatty alcohols such as oleyl alcohol and lauryl alcohol; monoglycerides of C8-C22 fatty acids such as tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; N-alkylpyrrolidone; and terpenes.

The composition of the invention comprises oleic acid as the penetration enhancer with the proviso of isopropyl myristate being excluded. In another embodiment, the penetration enhancer is a mixture of oleic acid and propylene glycol, with the proviso of isopropyl myristate being excluded.

In a preferred embodiment, penetration enhancers according to the present invention are introduced in a non-irritating and/or non-sensitizing amount. As used herein, the expression "non-irritating and/or non-sensitizing amount of at least one penetration enhancer" refers to amounts which the skilled person in the art would consider to be well-tolerated by the human skin, i.e., dermatologically acceptable. Using common general knowledge, the skilled person can determine non-irritating and/or non sensitizing amounts of penetration enhancer. In some embodiments, the non-irritating and/or non-sensitizing amount results in no detectable or sustained dermal adverse reaction (e.g., itching, reddening, burning sensation), or results in only a minimal reaction that is generally deemed to be acceptable by patients and health care providers.

In the invention, the penetration enhancer will be used in amount between 0.01% and 10% (w/w) of the composition, for example in an amount between 0.02% and 7%, between 0.05% and 5%, or between 0.1% and 1% (w/w) of the total composition.

**Gelling Agents**

As noted above, the compositions of the invention may optionally comprise at least one gelling agent.

As used herein, the term "gelling agent" specifies a compound, optionally of polymeric nature, having the capacity to form a gel when contacted with a specific solvent, such as water. Gelling agents (e.g., thickeners) are known in the art. Gelling agents may act to increase the viscosity of the pharmaceutical compositions of the invention. For example, a gelling agent may provide the composition with sufficient viscosity to allow easy application of the composition onto the skin. Additionally or alternatively, gelling agents may act as solubilizing agents.

Examples of gelling agents include anionic polymers such as acrylic acid based polymers (including polyacrylic acid polymers, such as CARBOPOL® by Noveon, Ohio), cellulose derivatives, poloxamers and poloxamines, more precisely, carbomers which are acrylic acid-based polymers, such as CARBOPOL ® 980 or 940, 981 or 941, 1342 or 1382, 5984, 934 or 934P (CARBOPOL ® are usually polymers of acrylic acid crosslinked with allyl sucrose or allylpentaerythritol), Ultrez, Pemulen TR1® or TR2®, Synthalen CR, etc.; cellulose derivatives such as carboxymethylcelluloses, hydroxypropylcelluloses (Klucel®), hydroxyethylcelluloses, ethylcelluloses, hydroxymethylcelluloses, hydroxypropylmethylcelluloses, and the like, and mixtures thereof; poloxamers or polyethylene-polypropylene copolymers such as Lutrol® grade 68 or 127, poloxamines and other gelling agents such as chitosan, dextran, pectins, and natural gums. Any one or more of these gelling agents may be used alone or in combination in the pharmaceutical compositions according to the invention. In one aspect, the gelling agent is selected from the group consisting of polyacrylic acids, cellulosics, and mixtures thereof.

The gelling agent will be used in an amount which does not exceed 5% (w/w) of the total composition, for example no more than 4%, 3%, 2%, 1%, or 0.5 % (w/w) of the total composition.

In one embodiment of the invention, the gelling agent will be used in an amount of at least 0.01%, 0.02%, 0.03%, 0.04%, 0.05%, 0.1%, or 0.2% (w/w) of the total composition.

In one embodiment of the invention, the gelling agent will be used in amount between 0.01% and 5% (w/w) of the composition, for example in an amount between 0.02% and 3%, between 0.05% and 2%, or between 0.1% and 1% (w/w) of the total composition.

## Moisturizers

The compositions of the invention may optionally comprise at least one moisturizer.

As used herein "moisturizer" specifies an agent that hydrates the skin. Moisturizers are known in the art. Moisturizers can be used either alone or in combination, including a combination of two or three (or more) different moisturizers.

In some embodiments, moisturizers are selected from emollients and/or humectants.

As used herein, "emollients" specify substances that soften the skin and tend to improve moisturization of the skin. Emollients are well known in the art, and include mineral oil, petrolatum, polydecene, isohexadecane, fatty acids and alcohols having from 10 to 30 carbon atoms; pelargonic, lauric, myristic, palmitic, stearic, isostearic, hydroxystearic, oleic, linoleic, ricinoleic, arachidic, behenic, and euricic acids and alcohols castor oil, cocoa butter, safflower oil, sunflower oil, jojoba oil, cottonseed oil, corn oil, olive oil, cod liver oil, almond oil, avocado oil, palm oil, sesame oil, squalene, Kikui oil, soybean oil, polyethylene glycol, wax esters, beeswax, spermaceti, silicone oils, dimethicones, and cyclomethicones. In some embodiments, the composition comprises one or more emollients that are liquid at room temperature.

As used herein "humectants" specifies hygroscopic substances that absorb water from the air. Humectants suitable for use in the invention include glycerine, propylene glycol, a polyol, sorbitol, maltitol, a polymeric polyol, polydextrose, quillaia, lactic acid, and urea.

Moisturizers suitable for use in the present invention may comprise amines, alcohols, glycols, amides, sulfoxides, and pyrrolidones. In one aspect, the moisturizer is selected from the group consisting of lactic acid, glycerine, propylene glycol, and urea.

Typically, the moisturizer will be used in an amount which does not exceed 30 % (w/w) of the total composition, for example no more than 20%, and for example no more than 15%, 10%, 8%, 7%, or 5% (w/w) of the total composition.

In one embodiment of the invention, the moisturizer will be used in an amount of at least 0.01%, 0.02%, 0.03%, 0.04%, 0.05%, 0.1%, 0.2%, or 0.5% (w/w) of the total composition.

In one embodiment of the invention, the moisturizer is used in amount between 0.01% and 30% (w/w) of the composition, for example in an amount between 0.05% and 20%, between 0.1% and 10%, or between 0.5% and 5% (w/w) of the total composition.

In one embodiment, the composition comprises between 0.01% and 30% (w/w) of glycerine, for example between 0.05% and 20%, between 0.1% and 10%, or between 0.5% and 5% (w/w) of glycerine.

## Aqueous vehicle

As noted above, the composition of the invention comprises an aqueous vehicle.

Aqueous vehicles are known in the art. According to one aspect of the invention, the aqueous vehicle comprises, in addition to water, ingredients useful in adjusting the pH, for instance at least one buffering agent. Buffering agents, including pharmaceutically acceptable buffering agents, are known in the art. In one aspect, the aqueous vehicle comprises at least one buffer, typically selected from the group consisting of citrate buffers, such as sodium citrate and/or potassium citrate; tris buffers, such as tris maleate; phosphate buffers, including Sorensen-type buffers, dibasic or monobasic phosphate, such as sodium dibasic or monobasic phosphate.

In another aspect, the pharmaceutical composition of the invention further comprises a base. Advantageously, the base is a pharmaceutically acceptable base, and may be selected from the group consisting of triethanolamine, sodium hydroxide, ammonium hydroxide, potassium hydroxide, arginine, aminomethylpropanol or tromethamine, and mixtures thereof. Where the pH of the pharmaceutical composition is not optimized for transdermal administration, such as where the gelling agent comprises at least one acrylic acid-based polymer, the use of a base contributes to the neutralization of the pharmaceutical composition, optimizing the composition for topical administration onto a skin surface. Furthermore, the use of the base (neutralizer) may allow for optimum swelling of the polymer chains during the neutralization of the charges and the formation of polymer salts. In embodiments where the gelling agent comprises an acrylic acid-based polymer, the base typically comprises triethanolamine. The use of a base also may allow optimal viscosity to be achieved. The skilled person will know how to choose a suitable amount of base for use in the composition, and may select the base based on the nature of the gelling agent present therein, and the alcohol content of the composition. For example, with carbomers and/or if there is a high alcohol content, one can use tromethamine and/or NaOH as a base, in amounts chosen so as to reach the desired final pH in the composition.

## Further Optional Components

The pharmaceutical compositions of the invention optionally may comprise other usual pharmaceutical additives, including salt(s), stabilizer(s), antimicrobial(s) such as paraben compounds, fragrance(s), and/or propellant(s).

Depending on the nature of the selected ingredients, it may be advantageous to include a surfactant. Surfactants are known in the art, and the skilled person can select suitable surfactants for use in the present invention, such as surfactants that are dermatologically and/or cosmetically acceptable.

Further examples include anionic surfactants such as SDS (sodium dodecyl sulphate), and the like, and cationic surfactants such as cetrimide (Alkyltrimethylammonium bromide) and the like.

Typically, surfactants will be used in the compositions of the invention in an amount which does not exceed 5% (w/w), for example no more than 4%, 3%, 2%, 1%, or 0.5% of the total composition.

In one embodiment of the invention, surfactants will be used in the compositions of the invention in an amount of at least 0.01%, for example at least 0.02%, 0.05%, or 0.1% of the total composition.

**Exemplary Compositions**

Exemplary, non-limiting compositions are provided below. As mentioned above, percentages (%) refer to amounts by weight based upon the total weight of the composition (w/w). The sum of the different components of the composition adds up to 100% (w/w) of the total composition.

In one embodiment, the composition of the invention comprises:

- (i) 3 to 6 % (w/w) of a SERM selected from the group consisting of endoxifen and the pharmaceutically acceptable salts thereof,
- (ii) 0.9 % (w/w) of Carbopol 980 (neutralized with 0.1 N NaOH),
- (iii) 0.5 % (w/w) of oleic acid,
- (iv) 67 % (w/w) of ethanol,
- (vi) q.s. 100 % (w/w) of water.

**Exemplary Modes of Administration**

The compositions may be administered by any means effective to apply the composition to a surface of the skin. For example, the compositions may be applied manually without or with an applicator such as a dropper or pipette, an applicator such as a swab, brush, cloth, pad, sponge, or with any other applicator, such as a solid support comprising paper, cardboard or a laminate material, including material comprising flocked, glued or otherwise fixed fibers. Alternatively, the compositions may be applied as an aerosol or non-aerosol spray, from a pressurized or non-pressurized container. In some embodiments, the compositions are administered in metered doses, such as from a metered dose applicator or from an applicator comprising a single dose of the composition.

**Devices**

One aspect of the invention provides a device for administering the compositions. In one embodiment, the device comprises a reservoir containing the composition and a topical applicator for applying the composition to a surface of the skin.

In one embodiment, the device is an opaque device which protects the composition of the invention from the adverse effects of light. Some of the SERMs are light-sensitive which deteriorate upon prolonged exposure to light.

The reservoir may be of any configuration and any material suitable for containing the composition. For example, the reservoir may be rigid or flexible, may be of a unitary construction (such as a molded material) or may be formed from different pieces secured together, such as by laminating, heat-sealing, gluing, welding, riveting, etc. For example, the reservoir may comprise a rolled wall, two walls substantially parallel joined at the vicinity of their periphery (where the walls may be, for example, flexible/deformable, formed by a thermoformed blister, or rigid), or a bottom wall and a cylindrical wall, or any other configuration suitable for containing the composition. In some embodiments, the reservoir comprises a bag, a pouch, a sachet, a blister, an ampoule, a pipette, a vial, a canister, or a bottle. In some embodiments, the reservoir comprises a deformable wall that is adapted to actuate flow of the composition when deformed such as those described in patent applications WO9527569, WO9615045 or US5630531. Deformable airtight reservoirs provide a good security against leakage of the composition and enable improved preservation of the composition, by protecting it from any atmospheric contact. In some embodiments, the reservoir is adapted to contain a single dose of the composition.

As used herein "topical applicator" specifies an applicator of any configuration and any material suitable for applying the composition to a surface of the skin. The topical applicator may be integrally formed with the reservoir, such that the reservoir and topical applicator comprise a unitary construction, or the topical applicator may be detachable from, or provided separately from, the reservoir.

For example, the topical applicator may comprise a dropper, pipette, swab, brush, cloth, pad, sponge, or any solid support, such as a support comprising paper, cardboard or a laminate material, including material comprising flocked, glued or otherwise fixed fibers. In some embodiments, the applicator is pre-loaded with composition, for example, the applicator may be impregnated with composition, such as with a unit dose of the composition. In other embodiments, the applicator is loaded with composition during use.

Alternatively, the topical applicator may comprise an aerosol or non-aerosol spray device, such as a hand pump.

In other embodiments, the topical applicator is an opening that permits the product to be dispensed therethrough. In some embodiments, the opening is provided with a removable and replaceable device for closing and opening the opening, such as a cap, stopper or plug, which can be placed within or over the opening such as by insertion, screwing, snapping, fitting, or otherwise. In another embodiment, the opening is provided with a removable and disposable device for opening the opening, such as any removable or secable, frangible, peelable or tearable covering over the opening. In other embodiments, the opening is provided with a nozzle or valve, such as a metered dose valve.

In some embodiments, the topical applicator is adapted to dispense a metered dose of the composition, such as a unit dose of a therapeutically effective amount of the composition. In some embodiments, the topical applicator is not a syringe, and the device does not comprise a syringe for intravenous administration.

In some embodiments, the device comprises a single reservoir. In other embodiments, the device contains two or more reservoirs, where each reservoir may contain a single dose of the composition, or may contain any amount of the composition. In some embodiments, the device comprises a single applicator for applying composition from two or more reservoirs. In other embodiments, the device comprises one applicator for applying composition from each reservoir.

In some embodiments, the invention provides a dose, unit dose, or multiple dose of the pharmaceutical composition, such as in a dose package, a unit dose package or a multiple dose package. In some embodiments, the packaging reflects a dosing regimen or schedule of application, such as daily, weekly, or twice weekly administration. Advantageously, such packaging of the pharmaceutical composition facilitates accurate application of an amount of the composition, such as a therapeutically effective amount.

According to one embodiment, the composition, device or packet is provided together with instructions for the use thereof in accordance with the methods described herein.

## Therapeutic Methods

The present invention also provides a pharmaceutical composition according to the invention for use in a method for treating a patient suffering from a breast disorder, comprising administering a therapeutically effective amount of the pharmaceutical composition.

The term 'treat' or 'treating' or 'treatment' as used herein refers to the administration of a therapeutically effective amount of a pharmaceutical composition according to the invention to a mammalian subject, including a human male or female, suffering from a condition, disorder, or disease. In accordance with some aspects of the invention, the administration may result in preventing the condition, disorder, or disease from occurring to a clinically diagnosable extent in a patient who may be predisposed to the condition, disorder, or disease, but not yet diagnosed as having the condition, disorder, or disease. In accordance with other aspects, the administration may result in inhibiting the condition, disorder, or disease, for example, arresting the development of the condition, disorder, or disease, relieving the condition, disorder, or disease, for example, causing regression of the condition, disorder, or disease, or relieving a condition caused by the disease or disorder, for example, stopping or reducing a symptom of the disease or disorder. Any such result may constitute the achievement of an intended therapeutic effect in a patient.

In one embodiment, the administration is performed by applying a therapeutically effective amount of the composition of the invention onto a surface of the skin of a patient in need thereof. In some embodiments, the patient to be treated is a mammal, such as a human. The patient may be a male or a female. In some embodiments, such as for the treatment of a breast condition or disorder, the composition is applied to a breast. In some other embodiments, such as for the treatment of a localized tumour, the composition is applied to the part of the skin where the tumour is located.

In some embodiments, the administration further comprises rubbing the composition into the patient's skin. This rubbing may comprise, for example, gentle rubbing of the composition onto the selected surface area, so that the composition substantially completely penetrates into the patient's skin.

The administration may follow any suitable administration regimen, as can be determined by those skilled in the art. For example, in one aspect, the method of the invention comprises once daily administration. In another aspect, the method comprises bi-weekly or once-weekly administration. Other suitable regimens are included within the scope of the invention.

The present invention also relates to the use of one of the above compositions for the manufacture of a medicament for treating a patient suffering from a breast disorder.

In particular, the pharmaceutical compositions, gels, packets and containers of the invention are useful for:

-  treating a patient suffering from or at risk of developing a breast disorder such as:

- conditions involving dense breast tissue. High density breast tissues are a predictor of breast cancer risk, and compromises mammographic sensitivity, which is a major issue for cancer detection and diagnostic. The dense breast tissue can be diffuse or nodular;

- benign breast diseases. Benign breast disease generally refers to a constellation of common non-malignant aberrations in breast tissue. These aberrations include numerous lesions that have well-defined histological characteristics, and can be classified as proliferative or nonproliferative. Exemplary benign breast diseases treatable by the present methods include adenosis, cysts, duct ectasia, fibroadenoma, fibrosis, hyperplasia, metaplasia and other fibrocystic changes. Each of these diseases, often referred to as "changes" or "conditions" due to their prevalence, have well-defined histological and clinical characteristics. "Adenosis" refers to generalized glandular disease of the breast. It typically involves an enlargement of breast lobules, which contain more glands than usual. In "sclerosing adenosis," or "fibrosing adenosis," the enlarged lobules are distorted by scarlike fibrous tissue. "Cysts" are abnormal sacs filled with fluid or semi-solid material, and lined by breast epithelial cells, developing from lobular structures. They begin as excess fluid inside breast glands, but may grow to proportions that stretch surrounding breast tissue, causing pain. "Fibrocysts" are cystic lesions circumscribed by, or situated within, a conspicuous amount of fibrous connective tissue. "Duct ectasia" refers to a dilation of mammary ducts by lipid and cellular debris. Rupture of the ducts induces infiltration by granulocytes and plasma cells. "Fibroadenoma" refers to benign tumors that are derived from glandular epithelium and contain a conspicuous stroma of proliferating fibroblasts and connective tissue. "Fibrosis" simply refers to a prominence of fibrous tissue in the breast. "Hyperplasia" refers to an overgrowth of cells, where several layers of cells line the basal membrane, without tumor formation. Hyperplasia increases the bulk of mammary tissue. In "epithelial hyperplasia," the cells lining breast ducts and lobules are involved, giving rise to the terms "ductal hyperplasia" and "lobular hyperplasia." Based on a histological determination, hyperplasia may be characterized as "usual" or "atypical". "Metaplasia" refers to a phenomenon in which a differentiated tissue of one type transforms into a differentiated tissue of another type. Metaplasia often results from an environmental change, and enables cells better to withstand the change;

- gynecomastia. Gynecomastia is a common clinical condition, often presenting secondarily to an underlying disorder, representing the benign and sometimes painful proliferation of breast tissue in young boys and adult males;

- breast cancer, especially non-invasive breast cancer;

- malignant melanoma;

- mastalgia. Mastalgia, also called "mastodynia" or breast pain, constitutes the most common breast problem for which women consult general medical practitioners. Its severity varies, but mastalgia can be so prolonged and intense as to interfere with normal daily activities, and even to disable afflicted individuals. Mastalgia can be classified according to three general sources of pain: (1) cyclical mammary pain, (2) non-cyclical mammary pain, and (3) extramammary pain. Cyclical mastalgia results from physiological breast enlargement, caused by estrogen-dependent vascular changes, during the luteal phase of the menstrual cycle, and affects a majority of premenopausal women. Cyclical mastalgia also can recur in postmenopausal women on estrogen replacement therapy, with a dose-dependent effect. "Non-cyclical mastalgia", as its name suggests, refers to pain in the breast that is not related to the menstrual cycle. A number of conditions give rise to non-cyclical mastalgia, including sclerosing adenosis, Tietz's syndrome and, rarely, breast cancer. Finally, extramammary mastalgia includes breast pain that is projected to the breast from other sources, as occurs, for example, when a patient feels pain from muscles or ribs that underlie the breasts.

- treating a patient suffering from localized cancer and/or tumours such as lung tumours;
- treating a bone-related disorder, comprising topically administering to a surface of skin of a patient in need thereof, a therapeutically effective amount of a pharmaceutical composition as described above. Said bone-related disorder may be selected from the group consisting of osteoporosis, menopause-associated osteoporosis, glucocorticoid-induced osteoporosis, Paget's disease, abnormal bone resorption, bone cancer, bone loss (generalized bone loss and/or localized bone loss), bone metastasis (with or without hypercalcemia), multiple myeloma and other conditions that feature bone fragility.

The present pharmaceutical compositions and gels can also be used in "combination therapy" with one or more further active agent(s).

In one embodiment, the present pharmaceutical compositions and gels are particularly suitable for treating patients for whom other therapies are not effective, or for treating women undergoing hormone replacement therapy (HRT).

## Methods of Making the Compositions

The invention also provides a process for preparing the pharmaceutical compositions of the invention, according to the appended claims. Those skilled in the art can prepare the pharmaceutical compositions of the invention by any suitable means, based on common general knowledge. For example, the SERM can be dissolved in the monoalcohol and mixed with the aqueous solvent, followed by addition of the other excipients, such as the moisturizer, and further mixing. A gelling agent, if present, can be introduced under stirring. A neutralizer, if present, usually is added at or near the end of the method, such as to the otherwise final composition. For example, if the composition comprises Carbopol, NaOH or triethanolamine can be used to neutralize the composition. Other optional components can be added at other stages of the method, in accordance with known procedures. For example, a preservative, if present, is added in an appropriate solvent, at any suitable time of the process.

## Examples

### Example 1: Endoxifen

HPLC Method

Endoxifen (mixture of two isomers) was analyzed by HPLC (Jasco, Japan, Model PU-2080 Plus pump and Model AS-2051 Plus autosampler) using UV detection. Chromatographic resolution was obtained on a C18 reverse-phase column (5 $\mu$m, 250 mm x 4.6 mm, HiQ sil, KYA Tec, Japan) with a guard column (ODS Hypersil 5 $\mu$m 10 x 4 mm, Thermo Electron Corporation, UK). The mobile phase was methanol:acetonitrile:water (350:350:300), 2 ml of acetic acid and 1 ml of triethanolamine. The mobile phase was filtered through 0.45 $\mu$m PVDF (Polyvinylidene Fluoride polymer) membrane filter. Injection volume was 50 $\mu$l and the flow rate was set at 0.7 ml/min. UV detection (Model UV-2975 Plus) was performed at 244 nm with a run time of 12 minutes. The retention times for Isomer I and Isomer II were 7.8 min. and 8.7 min., respectively. Each experiment was carried out in duplicate. Calibration used the external standard method. The calibration curve for endoxifen was established in the range 0.1 - 20 $\mu$g/mL. (The linearity interval for endoxifen; r2=0.9999).

Preparation of Stock Solutions

Standard solutions of endoxifen were prepared by dissolving 5 mg drug in 5 ml of ethanol. Dilutions were made with 3% cetrimide solution. Samples were filtered through 0.45 $\mu$m PVDF membrane filters.

Assay Accuracy and Precision

Accuracy of the analytical method was calculated from the percentage of the known added amount of analyte recovered in the samples ($\pm$CV). Accuracy of the HPLC method for endoxifen was assessed in triplicate at three concentrations (0.1, 2.5 and 20 $\mu$g/mL).
Accuracy of the HPLC method for endoxifen was 104.09% (CV: 5.69).
Precision was measured as the degree of repeatability of the analytical method. The precision was assessed in triplicate at three concentrations (0.1, 2.5 and 20 $\mu$g/mL).
Repeatability CV for endoxifen was 0.99%.

Stability of Endoxifen in analysis medium (Cetrimide solution, 3%)

The stability of endoxifen in the analysis medium (3% cetrimide solution) was determined. At various times during 24 hours, the concentration of endoxifen in the sample was measured. Endoxifen was stable in the analysis medium for 24 hours.

Specificity of Method

The specificity of the method was determined. A "sham" skin diffusion experiment was performed with no drug in the donor chamber. After 24 hours, the receptor phase was sampled and subjected to HPLC analysis using the above conditions. The goal was to determine if there were any absorption peaks originating from the skin or the from the cetrimide-containing receptor phase which might interfere with the analysis of the drug.

Solubility Studies

The saturated solubilities of endoxifen were determined in a mixture of absolute ethanol/water (70:30, w/w) by shaking an excess amount of drug in 1 ml of the co-solvent system for at least 24 hours at room temperature. The concentrations of the saturated solutions were determined by HPLC analysis after appropriate dilution.

Solubility of endoxifen in an ethanol:water system (70:30 w/w) and in cetrimide solution (3%, w/v) were 38.72 mg/mL (n=1), and 6.26± 0.28 mg/mL (n=3), respectively.

*In silico* prediction of skin permeation

Transdermal delivery of endoxifen was estimated *in silico* by calculating a maximum theoretical flux ($J_{max}$) across skin according to the following approach:

The maximum flux ($J_{max}$) at which a chemical can cross the skin is theoretically achieved when it is maintained in a saturated solution (or in neat chemical form) on the surface. The relevant equation that applies in these circumstances is Fick's 1$^{st}$ law:

$$J_{max} \cong \frac{D}{h} * K_{skin/vehicle} * C_{vehicle}^{sat} \qquad \textbf{(Equation 1)}$$

where $D$ is the chemical's diffusivity across the skin (typically that through the stratum corneum, the skin's least permeable and outermost layer), $h$ is the diffusion path-length, $K_{skin/vehicle}$ is the compound's partition coefficient between the skin and the vehicle contacting the surface, and $C_{vehicle}^{sat}$ is its saturation solubility in the vehicle. $K_{skin/vehicle}$ may be defined as follows:

$$K_{skin/vehicle} = \frac{C_{skin}^{sat}}{C_{vehicle}^{sat}} \qquad \textbf{(Equation 2)}$$

where $C_{skin}^{sat}$ and $C_{vehicle}^{sat}$ are the concentrations in skin and vehicle respectively. Equation 1 can then be reduced to a simpler form

$$J_{max} = \frac{D}{h} * C_{skin}^{sat} \qquad \textbf{(Equation 3)}$$

which shows that the maximum flux achievable across the barrier is independent of the formulation, providing that the formulation is saturated. That is, $J_{max}$ should be constant as long as the chemical is at its maximum thermodynamic activity in the vehicle (i.e., that it is saturated), and provided that the excipients in the formulation do not change the skin's barrier properties (e.g., exhibit permeation-enhancing or -retarding characteristics).

Hence, if the value of $J_{max}$ can be predicted from first principles, it should then be possible, with knowledge of the degree of saturation of the chemical in a particular formulation, to calculate the maximum amount absorbed across the skin following a specific scenario.

This objective can be achieved using an algorithm derived by Potts & Guy (Pharm Res. 1992, 9(5), 663-9) from an extensive database of the permeability coefficients of approximately 100 chemicals across human skin *in vitro* following their application in water. The permeability coefficient of a chemical ($K_p$) from an aqueous vehicle is defined as:

$$K_p = \frac{D * K_{skin/water}}{h} \qquad \textbf{(Equation 4)}$$

Multiple regression analysis of the experimental values of $K_p$ against various physicochemical variables led to the derivation of an equation (Potts & Guy 1992), which has been shown to have reasonable predictive power:

$$\log K_p = -2.7 + 0.71 * \log P - 0.0061 * MW \qquad \textbf{(Equation 5)}$$

where P is the octanol-water partition coefficient of the chemical and *MW* is its molecular weight.

In the above form, the units of permeability coefficient are cm/h. For very lipophilic chemicals, it is necessary to correct the value of $K_p$ calculated from Equation 5 to take into account the contribution of the living skin layers (viable epidermis and dermis) to the permeation process (Cleek and Bunge, Pharm Res, 1993, 10(4), 497-506):

$$K_p^{corr} = \frac{K_p}{1 + \dfrac{K_p \cdot \sqrt{MW}}{2.6}} \qquad \textbf{(Equation 6)}$$

Combining Equations 1, 2 and 6 yields:

$$J_{max} = K_p * C_{water}^{sat} \qquad \textbf{(Equation 7)}$$

The permeability coefficient can be calculated from Equations 5 and 6 and readily available physicochemical parameters (*MW* and *log P),* for which a very large database of values exists, or which can be calculated with many different approaches available on the internet. Equally, there is a considerable number of aqueous solubilities tabulated and/or accessible via the web.

Aqueous solubility $\left( C_{water}^{sat} \right)$ and the partition coefficient between octanol and water (logP) of endoxifen were estimated using the Osiris Property Explorer calculator (from Actelion's inhouse registration system). (http://www.chemexper.com).

According to this calculator, properties of a given molecule are predicted by a fragment-based approach by comparison with molecules for which those properties have been determined experimentally (see Balakin et al., Current Medicinal Chemistry, 2006, 13, p223-241, *"In Silico approaches to prediction of aqueous and DMSO solubility of drug-like compounds: trends, problems and solutions"* for a review on the in silico prediction of aqueous solubility of drug-like compounds).

The following results were obtained:

**Table 1**: Predicted transdermal flux for endoxifen using the Osiris property explorer estimates

| Compound | MW | Osiris predictions | | Predicted Jmax ($\mu$g/cm$^2$/h) |
|---|---|---|---|---|
| | | logP | $C_{water}^{sat}$ | |
| endoxifen | 373.5 | 5.33 | 8.90E-04 | 4.09E-02 |

The predicted maximum flux for endoxifen was 0.041 $\mu$g/cm$^2$/h which corresponds to a delivery of about 0.98 $\mu$g/cm$^2$ of drug in 24 hours.

The skilled person would be strongly dissuaded by this prediction from envisaging a transdermal delivery route for administering endoxifen, since he/she would expect poor results, and in particular, poor flux values. Thus, the *in silico* prediction severely undermines the feasibility of transdermal treatment with endoxifen.

Skin Permeation Study

Skin permeation studies were carried out using opaque side-by-side diffusion cells with an effective diffusion area of 0.71 cm$^2$. The receptor compartment had a volume of 3.2 ml and was maintained at $37\pm0.5°$C. The receptor solution was a 3% w/v cetrimide solution and allowed sink conditions to be maintained. The receptor phase was magnetically stirred at 100 rpm. Dermatomed abdominal pig skin (750 $\mu$m) was used. Skin was allowed to hydrate with isotonic saline solution for 1 h before the experiment was started. At t = 0, 3 ml of a saturated solution of endoxifen in 70:30 w/w ethanol/water was introduced into the donor chamber. Experiments were performed under complete occlusion. Post-application, at scheduled times (1, 9, 20 and 24 hours), 1 ml samples were taken from the receiver compartment and replaced with the same volume of fresh, temperature-equilibrated receptor fluid. Samples were filtered through 0.45 $\mu$m PVDF membrane filters. The amount of endoxifen in the samples was determined by HPLC using the method described above.

Table 2 shows the cumulative permeation ($\mu$g/cm$^2$) of endoxifen through abdominal pig skin from a saturated solution of the drug in 70:30 w/w ethanol-water. The endoxifen amount in the donor solution was 30.46 mg/mL.

**Table 2:**

| Time (hr) | Cell 1 $\mu$g/cm$^2$ | Cell 2 $\mu$g/cm$^2$ | Cell 3 $\mu$g/cm$^2$ | Cell 4 $\mu$g/cm$^2$ | Mean ($\pm$SD) $\mu$g/cm$^2$ 25 |
|---|---|---|---|---|---|
| 1 | - | - | - | - | - |
| 9 | 0.71 | 0.53 | - | - | 0.31$\pm$0.36 |
| 20 | 17.19 | 38.62 | 5.74 | 7.39 | 17.24$\pm$15.13 |
| 24 | 36.35 | 81.16 | 15.11 | 16.53 | 37.29$\pm$30.81 |
| (-) below the limit of detection (LOD) of 30ng/ml. | | | | | |

Therefore, the experimentally determined maximum flux observed for endoxifen after 24 hours was around 37.29 $\mu$g/cm$^2$. This value is more than one order of magnitude higher (40 times higher) than that predicted by the *in silico* calculations set forth above 4.09E-02 $\mu$g/cm$^2$/h, which corresponds to the delivery of about 0.98 $\mu$g/cm$^2$ of drug in 24 hours.

**Example 2 (not according to the invention): Droloxifene citrate**

Similar experiments were conducted for droloxifene citrate.

*In silico* prediction of skin permeation

Table 3 sets forth the predicted transdermal flux for droloxifene citrate using the Osiris property explorer estimates, as described above for endoxifen.

Table 3:

| Compound | MW | Osiris predictions | | Predicted Jmax ($\mu$g/cm$^2$/h) |
|---|---|---|---|---|
| | | logP | $C_{water}^{sat}$ | |
| droloxifene citrate | 387.52 | 6.15 | 3.19E-03 | 2.63E-01 |

The predicted maximum flux for droloxifene citrate was 0.263 $\mu$g/cm$^2$/h which corresponds to a delivery of about 6.3 $\mu$g/cm$^2$ of drug in 24 hours.

Validation of HPLC Method

Droloxifene citrate was analyzed by HPLC (Jasco, Japan, Model PU-2080 Plus pump and Model AS-2051 Plus autosampler) using UV detection. Chromatographic resolution was obtained on a C18 reverse-phase column (5 $\mu$m, 250 mm x 4.6 mm, HiQ sil, KYA Tec, Japan). The mobile phase was methanol:acetonitrile:water (350:350:300), 2 ml of acetic acid and 1 ml of triethanolamine. The mobile phase was filtered through 0.45 $\mu$m PVDF membrane filter. Injection volume was 50 $\mu$l and the flow rate was set at 1 ml/min. UV detection (Model UV-2975 Plus) was performed at 235 nm with a run time of 10 minutes. The retention time was 6.1 min. Each experiment was carried out in duplicate. Calibration used the external standard method. The calibration curve for droloxifene citrate was established in the range 0.1 - 20 $\mu$g/mL. (The linearity interval for droloxifene citrate ; r2=0.9999)

Assay Accuracy and Precision

Accuracy of the analytical method was calculated from the percentage of the known added amount of analyte recovered in the samples ($\pm$CV). Accuracy of the HPLC method for droloxifene citrate was assessed in triplicate at three concentrations (0.1, 5 and 10 $\mu$g/mL).

Accuracy of the HPLC method for droloxifene citrate was 99.12% (CV: 2.6)

Precision was measured as the degree of repeatability of the analytical method. The precision was assessed in triplicate at three concentrations (0.1, 5 and 10 $\mu$g/mL).

Repeatability CV for droloxifene citrate was 0.58%

Stability of Droloxifene citrate in analysis medium (Cetrimide solution, 3%)

Droloxifene citrate was stable in the analysis medium for 24 hours.

Solubility Studies

Solubility of droloxifene citrate in the mixture of ethanol:water (70:30; w/w) and cetrimide solution (3%, w/v) were 47.45±1.70 mg/mL and 8.9±0.2 mg/ mL, respectively (n=2).

Skin Permeation Study

Table 4 shows the cumulative permeation ($\mu$g/cm$^2$) of droloxifene citrate through abdominal pig skin from a saturated solution of the drug in 70:30 w/w ethanol-water. The droloxifene citrate amount in the donor solution was 45.93 ±1.15 mg/mL.

**Table 4:**

| Time (h) | Cell 1 $\mu$g/cm$^2$ | Cell 2 $\mu$g/cm$^2$ | Cell 3 $\mu$g/cm$^2$ | Cell 4 $\mu$g/cm$^2$ | Cell 5 $\mu$g/cm$^2$ | Cell 6 $\mu$g/cm$^2$ | Mean (±SD) $\mu$g/cm$^2$ |
|---|---|---|---|---|---|---|---|
| 1 | - | - | - | - | - | - | - |
| 9 | - | - | - | - | - | - | - |
| 20 | 0.38 | -* | 1.12 | 0.69 | -* | 0.30 | 0.62 ± 0.37 |
| 24 | 0.95 | -* | 2.36 | 1.43 | -* | 0.58 | 1.33 ± 0.77 |
| *The amounts are lower than quantitation limit of analysis method. | | | | | | | |

The *in silico* predicted maximum steady-state flux of droloxifene citrate was 0.263 $\mu$g/cm$^2$/hr, which corresponds to the delivery of about 6.3 $\mu$g/cm$^2$ of drug in 24 hours. The experimental results indicate a lower delivery in 24 hours of 1.33 $\mu$g/cm$^2$.

**Example 3 (not according to the invention): Clomifene citrate**

Similar experiments were conducted for clomifene citrate.

*In silico* prediction of skin permeation

Table 5 shows the predicted transdermal flux for clomifene citrate using the Osiris property explorer estimates, as discussed above for endoxifen.

**Table 5:**

| Compound | MW | Osiris predictions | | Predicted Jmax ($\mu$g/cm$^2$/h) |
|---|---|---|---|---|
| | | logP | $C^{sat}_{water}$ | |
| clomifene citrate | 405.97 | 7.15 | 3.80E-04 | 4.27E-02 |

Validation of HPLC Method

Clomifene citrate (mixture of Isomers E and Z) was analyzed by HPLC (Jasco, Japan, Model PU-2080 Plus pump and Model AS-2051 Plus autosampler) using UV detection. Chromatographic resolution was obtained on a C18 reverse-phase column (5 $\mu$m, 250 mm x 4.6 mm, HiQ sil, KYA Tec, Japan). The mobile phase was methanol:water:trifluoroacetic acid (75:25:0.1). The mobile phase was filtered through 0.45 $\mu$m PVDF membrane filter. Injection volume was 50 $\mu$l and the flow rate was set at 0.6 ml/min. UV detection (Model UV-2975 Plus) was performed at 235 nm with a run time of 30 minutes. The retention times for the two isomers were 16.6 and 19.0 min. Each experiment was carried out in duplicate. Calibration used the external standard method. The calibration curve for clomifene citrate was established in the range 0.25 - 20 $\mu$g/mL. (The linearity interval for clomifene citrate was; r2=0.9999)

Assay Accuracy and Precision

Accuracy of the analytical method was calculated from the percentage of the known added amount of analyte recovered in the samples ($\pm$CV). Accuracy of the HPLC method for clomifene citrate was assessed in triplicate at three concentrations (0.25, 2.5 and 20 $\mu$g/mL).
Accuracy of the HPLC method for clomifene citrate was 99.0% (CV: 3.18).
Precision was measured as the degree of repeatibility of the analytical method. The precision was assessed in triplicate at three concentrations (0.25, 2.5 and 20 $\mu$g/mL).
Repeatability CV for clomifene citrate was 0.56%.

Stability of clomifene citrate in analysis medium (Cetrimide solution, 3%)

Clomifene citrate was stable in the analysis medium for 24 hours.

Solubility Studies

The saturated solubilities of clomifene citrate were determined in a series of ethanol/water mixtures (40:60, 50:50, 60:40 and 70:30, w/w) by shaking an excess amount of drug in 0.6 ml of the co-solvent system for at least 24 hours at room temperature. The solubility of drug was also determined in the 3% cetrimide solution. The concentrations of the saturated solutions were determined by HPLC analysis after appropriate dilution.
Saturated solubilities of clomifene citrate in a series of ethanol/water mixtures (n=3) are set forth in Table 6.

**Table 6:**

| EtOH : Water (w/w) | Expt 1 (mg/ml) | Expt 2 (mg/ml) | Expt 3 (mg/ml) | Average ($\pm$SD) conc (mg/ml) |
|---|---|---|---|---|
| 40:60 | 20.97 | 21.02 | 20.98 | 20.99$\pm$0.03 |
| 50:50 | 35.36 | 33.36 | 35.81 | 34.84$\pm$1.30 |
| 60:40 | 39.05 | 40.88 | 40.15 | 40.03$\pm$0.92 |
| 70:30 | 42.64 | 40.90 | 41.69 | 41.74$\pm$0.87 |

Solubility of clomifene citrate in 3% cetrimide solution was 24.76$\pm$ 1.99 mg/ml.

Skin Permeation Study

Table 7 shows the cumulative permeation ($\mu$g/cm$^2$) of clomiphene citrate through abdominal pig skin from a saturated solution of the drug in 70:30 w/w ethanol-water.

**Table 7:**

| Time (h) | Cell 1 $\mu$g/cm$^2$ | Cell 2 $\mu$g/cm$^2$ | Cell 3 $\mu$g/cm$^2$ | Cell 4 $\mu$g/cm$^2$ | Cell 5 $\mu$g/cm$^2$ | Cell 6 $\mu$g/cm$^2$ | Mean $\pm$ SD ($\mu$g/cm$^2$) |
|---|---|---|---|---|---|---|---|
| 1 | - | - | - | - | - | - | - |
| 9 | - | - | - | - | - | - | - |
| 20 | 0.94 | - | 1.20 | - | 1.18 | 3.62 | 1.74$\pm$1.26 |

(continued)

| Time (h) | Cell 1 $\mu$g/cm$^2$ | Cell 2 $\mu$g/cm$^2$ | Cell 3 $\mu$g/cm$^2$ | Cell 4 $\mu$g/cm$^2$ | Cell 5 $\mu$g/cm$^2$ | Cell 6 $\mu$g/cm$^2$ | Mean $\pm$ SD ($\mu$g/cm$^2$) |
|---|---|---|---|---|---|---|---|
| 24 | 1.73 | 0.56 | 2.31 | 0.61 | 2.55 | 6.78 | 2.42 $\pm$ 2.29 |

The *in silico* predicted maximum steady-state flux of clomifene citrate was 0.043 $\mu$g/cm$^2$/hr, which corresponds to the delivery of about 1 $\mu$g/cm$^2$ of drug in 24 hours.

The experimental results for the citrate salt from an ethanol/water solution indicate a higher value of 2.42 $\mu$g/cm$^2$.

**Example 4 (not according to the invention): Tamoxifene citrate**

Similar experiments were conducted for tamoxifen citrate.

*In silico* prediction of skin permeation

Table 8 shows the predicted transdermal flux for tamoxifen citrate using the Osiris property explorer estimates, as discussed above fore endoxifen.

**Table 8:**

| Compound | MW | Osiris predictions | | Predicted Jmax ($\mu$g/cm$^2$/h) |
|---|---|---|---|---|
| | | logP | $C_{water}^{sat}$ | |
| tamoxifen citrate | 371.52 | 6.82 | 1.08E-03 | 1.25E-01 |

Validation of HPLC Method

Tamoxifen citrate was analyzed by HPLC (Jasco, Japan, Model PU-2080 Plus pump and Model AS-2051 Plus autosampler) using UV detection. Chromatographic resolution was obtained on a C18 reverse-phase column (5 $\mu$m, 250 mm x 4.6 mm, HiQ sil, KYA Tec, Japan). The mobile phase was methanol:water:trifluoroacetic acid (75:25:0.1). The mobile phase was filtered through 0.45 $\mu$m PVDF membrane filter. Injection volume was 50 $\mu$l and the flow rate was set at 0.6 ml/min. UV detection (Model UV-2975 Plus) was performed at 237 nm with a run time of 30 minutes. The retention time for tamoxifene citrate was 22.0 min. Each experiment was carried out in duplicate. Calibration used the external standard method. The calibration curve for tamoxifen citrate was established in the range 0.1 - 20 $\mu$g/mL. (The linearity interval for tamoxifen citrate was; r2=0.9999).

Assay Accuracy and Precision

Accuracy of the analytical method was calculated from the percentage of the known added amount of analyte recovered in the samples ($\pm$CV). Accuracy of the HPLC method for tamoxifen citrate was assessed in triplicate at three concentrations (0.1, 2.5 and 20 $\mu$g/mL).

Accuracy of the HPLC method for tamoxifen citrate was 102.12% (CV: 4.98).

Precision was measured as the degree of repeatability of the analytical method. The precision was assessed in triplicate at three concentrations (0.1, 0.25 and 20 $\mu$gmL).

Repeatability CV for tamoxifen citrate was 0.80%.

Stability of tamoxifen citrate in analysis medium (Cetrimide solution, 3%)

Tamoxifen citrate was stable in the analysis medium for 24 hours.

Solubility Studies

The saturated solubilities of tamoxifen citrate were determined in a series of ethanol/water mixtures (40:60, 50:50, 60:40 and 70:30, w/w) by shaking an excess amount of drug in 1 ml of the co-solvent system for at least 48 hours at room temperature. The solubility of drug was also determined in the 3% cetrimide solution. The concentrations of the saturated solutions were determined by HPLC analysis after appropriate dilution.

Saturated solubilities of tamoxifen citrate in a series of ethanol/water mixtures (n=3) are set forth in Table 9.

**Table 9:**

| EtOH : Water (w/w) | Expt 1 (mg/ml) | Expt 2 (mg/ml) | Expt 3 (mg/ml) | Average ($\pm$SD) conc (mg/ml) |
|---|---|---|---|---|
| 40:60 | 19.44 | 19.06 | 18.93 | 19.15$\pm$0.26 |
| 50:50 | 31.59 | 31.19 | 29.12 | 30.63$\pm$1.33 |
| 60:40 | 40.51 | 40.70 | 41.35 | 40.85$\pm$0.44 |
| 70:30 | 43.10 | 43.65 | 41.45 | 42.73$\pm$1.15 |

Solubility of tamoxifen citrate in 3% cetrimide solution was 3.126$\pm$ 0.064 mg/mL.

Skin Permeation Study

Table 10 shows the cumulative permeation ($\mu$g/cm$^2$) of tamoxifen citrate through abdominal pig skin from a saturated solution of the drug in 70:30 w/w ethanol-water.

**Table 10:**

| Time (h) | Cell 1 $\mu$g/cm$^2$ | Cell 2 $\mu$g/cm$^2$ | Cell 3 $\mu$g/cm$^2$ | Cell 4 $\mu$g/cm$^2$ | Cell 5 $\mu$g/cm$^2$ | Cell 6 $\mu$g/cm$^2$ | Mean ($\pm$SD) $\mu$g/cm$^2$ |
|---|---|---|---|---|---|---|---|
| 1 | - | - | - | - | - | - | - |
| 9 | - | - | - | - | - | - | - |
| 20 | 0.58 | 3.33 | 1.05 | 2.41 | 1.06 | 2.51 | 1.83$\pm$1.08 |
| 24 | 1.05 | 5.83 | 2.05 | 4.19 | 1.86 | 4.85 | 3.30$\pm$1.92 |

The *in silico* predicted maximum steady-state flux of tamoxifen was about 0.125 $\mu$g/cm$^2$/hr, which corresponds to the delivery of about 3 $\mu$g/cm$^2$ of drug in 24 hours. The experimental results for the citrate salt from an ethanol/water solution indicate a value of 3.30 $\mu$g/cm$^2$ which is very close to this prediction.

**Example 5 (not according to the invention): 4-hydroxy tamoxifen**

Similar experiments were conducted for 4-OH Tamoxifen.

*In silico* prediction of skin permeation

Table 11 shows the predicted transdermal flux for 4-OH tamoxifen using the Osiris property explorer estimates, as discussed above for endoxifen.

**Table 11:**

| Compound | MW | Osiris predictions | | Predicted Jmax ($\mu$g/cm$^2$/h) |
|---|---|---|---|---|
| | | logP | $C_{water}^{sat}$ | |
| 4-OH Tamoxifen | 387.52 | 5.91 | 2.99E-03 | 2.09E-01 |

Validation of HPLC Method

4-OH Tamoxifen (E/Z isomers 50:50) was analyzed by HPLC (Jasco Model PU-2080 Plus pump and Model AS-2051 Plus autosampler) using UV detection. Chromatographic resolution was obtained on a C18 reverse-phase column (5 $\mu$m, 250 mm x 4.6 mm, HiQ sil, KYA Tec, Japan). The mobile phase was methanol:acetonitrile:water (350:350:300), 2 ml of acetic acid and 1 ml of triethanolamine (pH 5.5$\pm$0.05). The mobile phase was filtered through

0.45 $\mu$m PVDF membrane filter. Injection volume was 50 $\mu$l and the flow rate was set at 1 ml/min. The UV detector (Model UV-2975 Plus) was set at 243 nm and run time was 10 min. The retention times for Isomer I and Isomer II were 5.7 and 6.3 min., respectively. Each experiment was carried out in duplicate. Calibration used the external standard method. Calibration curves for Isomer I and Isomer II were established in the range 0.25 - 50 $\mu$g/mL. (The linearity interval for 4-OH Tamoxifen was; r2=0.9999).

The HPLC analysis method separated the two isomer peaks with high resolution at all concentrations.

Assay Accuracy and Precision

Accuracy of the analytical method was calculated from the percentage of the known added amount of analyte recovered in the samples ($\pm$CV). Accuracy of the HPLC method for Isomers I and II was assessed in triplicate at two concentrations (0.25 $\mu$g/mL and 20 $\mu$g/mL).

Accuracy of the HPLC method (i.e., recoveries) for Isomers I and II were 98.42% (CV: 0.78) and 98.13% (CV: 0.87), respectively.

Precision was measured as the degree of repeatibility of the analytical method. The precision was assessed in triplicate at two concentrations (0.25 $\mu$g/mL and 20 $\mu$g/mL ).

Repeatability CVs for Isomers I and II were 0.78% and 0.87%, respectively.

Stability of 4-OH Tamoxifen in analysis medium (Cetrimide solution, 3%)

4-OH Tamoxifen (Isomers I and II) was stable in the analysis medium for 30 hours.

Solubility Studies

The saturated solubilities of 4-OH Tamoxifen were determined in a series of ethanol/water mixtures (40:60, 50:50, 60:40 and 70:30, w/w) by shaking an excess amount of drug in 1 ml of the co-solvent system for at least 24 hours at room temperature. The solubility of drug was also determined in the 3% cetrimide solution. The concentrations of the saturated solutions were determined by HPLC analysis after appropriate dilution.

Saturated solubilities of 4-OH Tamoxifen (total amount of Isomer I + Isomer II) in a series ofethanol/water mixtures are set forth in Table 12 (n=3).

**Table 12:**

| EtOH : Water (w/w) | Expt 1 ($\mu$g/ml) | Expt 2 ($\mu$g/ml) | Expt 3 ($\mu$g/ml) | Average concentration$\pm$SD ($\mu$g/ml) |
|---|---|---|---|---|
| 40:60 | 481 | 309 | 282 | 357 $\pm$ 108 |
| 50:50 | 1101 | 1224 | 1228 | 1184 $\pm$ 72 |
| 60:40 | 3763 | 3642 | 3603 | 3669 $\pm$ 83 |
| 70:30 | 8243 | 8390 | 8181 | 8271 $\pm$ 108 |

Solubility of 4-OH Tamoxifen (total amount of Isomer I + Isomer II) in 3% cetrimide solution was determined to be 1431 $\pm$ 105 $\mu$g/mL.

Skin Permeation Study

Table 13 shows the cumulative permeation ($\mu$g/cm$^2$) of 4-OH Tamoxifen through abdominal pig skin from a saturated solution of the drug in 70:30 v/v ethanol-water.

**Table 13:**

| Time (hr) | Cell 1 | Cell 2 | Cell 3 | Cell 4 | Cell 5 | Cell 6 | Cell 7 | Cell 8 | Cell 9 | Cell 10 | Average ($\pm$ SD) |
|---|---|---|---|---|---|---|---|---|---|---|---|
| 3 | - | - | - | - | - | - | - | - | - | - | - |
| 6 | - | - | - | - | - | - | - | - | - | - | - |
| 9 | 4.1 | 3.3 | - | - | - | - | - | - | - | - | - |

(continued)

| Time (hr) | Cell 1 | Cell 2 | Cell 3 | Cell 4 | Cell 5 | Cell 6 | Cell 7 | Cell 8 | Cell 9 | Cell 10 | Average ($\pm$ SD) |
|---|---|---|---|---|---|---|---|---|---|---|---|
| 20 | 19.0 | 9.1 | 5.7 | 4.2 | 4.3 | 6.1 | 4.1 | 12.3 | 8.0 | 9.8 | 8.3 $\pm$ 4.7 |
| 24 | 27.3 | 12.1 | 9.0 | 6.3 | 7.8 | 9.7 | 7.9 | 18.1 | 12.2 | 14.3 | 12.5 $\pm$ 6.3 |

The *in silico* predicted maximum steady-state flux was 0.25 $\mu$g/cm$^2$/hr, which corresponds to the delivery of about 6 $\mu$g/cm$^2$ of drug in 24 hours. The experimental results for the 4-OH tamoxifen from an ethanol/water solution indicate a higher value of 12.5 $\mu$g/cm$^2$.

**Example 6 (not according to the invention): Toremifene**

Similar experiments were conducted for toremifene.

*In silico* prediction of skin permeation

Table 14 shows the predicted transdermal flux for toremifene using the Osiris property explorer estimates, as discussed above for endoxifen.

**Table 14:**

| Compound | MW | Osiris predictions | | Predicted Jmax ($\mu$g/cm$^2$/h) |
|---|---|---|---|---|
| | | logP | $C^{sat}_{water}$ | |
| toremifene | 405.97 | 6.53 | 4.00E-04 | 3.67E-02 |

Validation of HPLC Method

Toremifene was analyzed by HPLC (Jasco Model PU-2080 Plus pump and Model AS-2051 Plus autosampler) using UV detection. Chromatographic resolution was obtained on a C18 reverse-phase column (5 $\mu$m, 250 mm x 4.6 mm, HiQ sil, KYA Tec, Japan). The mobile phase was acetonitrile: methanol:water (31:50:19) and 0.35 ml of trifluoroacetic acid. The mobile phase was filtered through 0.45 $\mu$m PVDF membrane filter. Injection volume was 50 $\mu$l and the flow rate was set at 0.5 ml/min. UV detection (Model UV-2975 Plus) was performed at 237 nm with a run time of 13 minutes. The retention time for toremifene was 9.2 min. Each experiment was carried out in duplicate. Calibration used the external standard method. The calibration curve for toremifene was established in the range 0.1 - 10 $\mu$g/mL. (The linearity interval for toremifene was; r2=0.9999).

Assay Accuracy and Precision

Accuracy of the analytical method was calculated from the percentage of the known added amount of analyte recovered in the samples ($\pm$CV). Accuracy of the HPLC method for toremifene was assessed in duplicate at three concentrations (0.1, 0.25 and 10 $\mu$g/mL).
Accuracy of the HPLC method for toremifene was 97.77% (CV: 2.77).
Precision was measured as the degree of repeatibility of the analytical method. The precision was assessed in duplicate at three concentrations (0.1, 0.25 and 10 $\mu$g/mL).
Repeatability CV for toremifene was 0.86%.

Stability of toremifene in analysis medium (Cetrimide solution, 3%)

Toremifene was stable in the analysis medium for 24 hours.

Solubility Studies

The saturated solubilities of toremifene were determined in a series of ethanol/water mixtures (40:60, 50:50, 60:40 and 70:30, w/w) by shaking an excess amount of drug in 1 ml of the co-solvent system for at least 24 hours at room temperature. The solubility of drug was also determined in the 3% cetrimide solution. The concentrations of the saturated solutions were determined by HPLC analysis after appropriate dilution.

Saturated solubilities of toremifene in a series of ethanol/water mixtures are set forth in Table 15 (n=3).

**Table 15:**

| EtOH : Water (w/w) | Expt 1 ($\mu$g/ml) | Expt 2 ($\mu$g/ml) | Expt 3 ($\mu$g/ml) | Average concentration$\pm$SD ($\mu$g/ml) |
|---|---|---|---|---|
| 40:60 | 74.8 | 75.4 | 74 | 74.7 $\pm$ 1.1 |
| 50:50 | 350 | 333 | 324 | 335 $\pm$ 15 |
| 60:40 | 1088 | 1063 | 1009 | 1054 $\pm$ 37 |
| 70:30 | 2861 | 2698 | 2623 | 2727 $\pm$ 124 |

Solubility of toremifene in 3% cetrimide solution was determined to be 302$\pm$14 $\mu$g/mL.

Skin Permeation Study

Table 16 shows the cumulative permeation ($\mu$g/cm$^2$) of toremifene through abdominal pig skin from a saturated solution of the drug in 70:30 w/w ethanol-water.

**Table 16**

| Time (hr) | Cell 1 | Cell 2 | Cell 3 | Cell 4 | Cell 5 | Mean $\pm$ SD |
|---|---|---|---|---|---|---|
| 1 | - | - | - | 3.38 | - | 3.38 |
| 9 | - | 0.25 | 0.52 | 5.35 | - | 1.53 $\pm$ 2.6 |
| 20 | 2.03 | 7.83 | 3.02 | 6.55 | - | 3.89 $\pm$ 3.2 |
| 24 | 3.35 | 9.65 | 4.30 | 7.47 | 0.62 | 5.08 $\pm$ 3.5 |

The *in silico* predicted maximum steady-state flux of toremifene was about 0.037 $\mu$g/cm$^2$/hr, which corresponds to the delivery of about 0.9 $\mu$g/cm$^2$ of drug in 24 hours. The experimental results indicate a higher delivery of about 5.08 $\mu$g/cm$^2$.

These six examples indicate that the experimentally determined maximum flux for each SERM was not uniformly correlated with the predicted flux obtained by the *in silico* calculations. Instead, the predicted maximum flux for the SERMS was lower, higher, or comparative to the maximum flux observed experimentally, depending on the SERM.

Therefore, the skilled person would not have been able to predict from *in silico* calculations which SERM(s) would be appropriate for administering via a transdermal delivery route, because the maximum flux achieved experimentally could not be predicted. Indeed, the *in silico* prediction severely undermines the feasibility of transdermal delivery of endoxifen, clomifene and toremifene, although the experimental results indicate that such transdermal delivery is indeed feasible. Out of these SERMs, endoxifene, due to its high maximum flux observed experimentally, is the more promising SERM for transdermal application, and, for this reason, it has been selected in the present invention. Indeed, for a transdermal application, it is important that the SERM has high flux permeation as a less important amount of the composition will be needed to be applied to the skin in order to deliver a therapeutically effective amount of SERM to the tissues.

**Claims**

1. A transdermal pharmaceutical composition for transdermal administration to a skin surface wherein said composition comprises:

   (i) 0.01 to 10 % (w/w), preferably 2 to 5 % (w/w) of endoxifen or a pharmaceutically acceptable salt thereof,
   (ii) 60 to 80 % (w/w) of at least one monoalcohol, wherein the at least one monoalcohol is selected from $C_2$-$C_4$ monoalcohols, preferably, ethanol or isopropanol,
   (iii) 0.01 to 10 % (w/w) of at least one penetration enhancer, wherein the at least one penetration enhancer is oleic acid and with the proviso of isopropyl myristate being excluded,
(iv) 0 to 5 % (w/w) of at least one gelling agent, preferably, polyacrylic acids, cellulosics, or mixtures thereof, and
(v) q.s. 100 % (w/w) aqueous vehicle.

2. A transdermal pharmaceutical composition according to claim 1, wherein the at least one penetration enhancer is a mixture of oleic acid and propylene glycol.

3. A transdermal pharmaceutical composition according to claim 1, for use in a method of administering a therapeutically effective amount of endoxifen or the pharmaceutically acceptable salt thereof, to a patient in need thereof, comprising transdermally administering the said composition to a surface of skin of said patient.

4. A transdermal pharmaceutical composition for use according to claim 3, wherein the patient is suffering from a breast disorder.

5. A transdermal pharmaceutical composition for use according to claim 3, wherein the patient is suffering from estrogen imbalance.

6. A transdermal pharmaceutical composition for use according to claim 5, wherein the estrogen imbalance is a breast disorder selected from benign breast diseases, gynecomastia, breast cancer, mastalgia and conditions involving dense breast tissue.

7. A dose packet, unit dose packet or multiple dose packet containing a transdermal pharmaceutical composition according to claim 1.

8. A dispenser, optionally with hand pump, containing a transdermal pharmaceutical composition according to claim 1.

9. A process for preparing a transdermal pharmaceutical composition according to claim 1 comprising the steps of preparing a mixture comprising:

(i) at least one monoalcohol selected from $C_2$-$C_4$ monoalcohols,
(ii) endoxifen or the pharmaceutically acceptable salts thereof,
(iii) an aqueous vehicle,
(iv) at least one penetration enhancer, wherein the at least one penetration enhancer is oleic acid and with the proviso of isopropyl myristate being excluded, and
(v) optionally, at least one gelling agent.

**Patentansprüche**

1. Transdermale pharmazeutische Zusammensetzung zur transdermalen Verabreichung an eine Hautoberfläche, wobei die Zusammensetzung umfasst:

(i) 0,01 bis 10% (w/w), bevorzugt 2 bis 5% (w/w) Endoxifen oder ein pharmazeutisch annehmbares Salz desselben,
(ii) 60 bis 80% (w/w) wenigstens eines Monoalkohols, wobei der wenigstens eine Monoalkohol ausgewählt ist aus $C_2$-$C_4$-Monoalkoholen, bevorzugt Ethanol oder Isopropanol,
(iii) 0,01 bis 10% (w/w) wenigstens eines Penetrationsverbesserers, wobei der wenigstens eine Penetrationsverbesserer Oleinsäure ist, und mit der Maßgabe, dass Isopropylmyristat ausgeschlossen ist,
(iv) 0 bis 5% (w/w) wenigstens eines Geliermittels, bevorzugt Polyacrylsäuren, Cellulosestoffe oder Mischungen derselben, und
(v) q.s. 100% (w/w) wässriger Trägerstoff.

2. Transdermale pharmazeutische Zusammensetzung nach Anspruch 1, wobei der wenigstens eine Penetrationsverbesserer eine Mischung aus Oleinsäure und Propylenglykol ist.

3. Transdermale pharmazeutische Zusammensetzung nach Anspruch 1, zur Verwendung in einem Verfahren zum Verabreichen einer therapeutisch wirksamen Menge von Endoxifen oder dem pharmazeutisch annehmbaren Salz desselben, an einen Patienten, der dieses bedarf, umfassend transdermales Verabreichen der Zusammensetzung auf eine Oberfläche der Haut des Patienten.

**4.** Transdermale pharmazeutische Zusammensetzung zur Verwendung nach Anspruch 3, wobei der Patient unter einer Brusterkrankung leidet.

**5.** Transdermale pharmazeutische Zusammensetzung zur Verwendung nach Anspruch 3, wobei der Patient an einem Östrogenungleichgewicht leidet.

**6.** Transdermale pharmazeutische Zusammensetzung zur Verwendung nach Anspruch 5, wobei das Östrogenungleichgewicht eine Brusterkrankung ist, ausgewählt aus gutartigen Brusterkrankungen, Gynäkomastie, Brustkrebs, Mastalgie und Zustände, die dichtes Brustgewebe einschließen.

**7.** Dosispaket, Einheitsdosispaket oder Mehrfachdosispaket enthaltend eine transdermale pharmazeutische Zusammensetzung nach Anspruch 1.

**8.** Dispenser, optional mit Handpumpe, enthaltend eine transdermale pharmazeutische Zusammensetzung nach Anspruch 1.

**9.** Verfahren zum Herstellen einer transdermalen pharmazeutischen Zusammensetzung nach Anspruch 1, umfassend die Schritte eines Herstellens einer Mischung, umfassend:

(i) wenigstens einen Monoalkohol ausgewählt aus $C_2$-$C_4$-Monoalkoholen,
(ii) Endoxifen oder das pharmazeutisch annehmbare Salz desselben,
(iii) einen wässrigen Trägerstoff,
(iv) wenigstens einen Penetrationsverbesserer, wobei der wenigstens eine Penetrationsverbesserer Oleinsäure ist, mit der Maßgabe, dass Isopropylmyristat ausgeschlossen ist, und
(v) optional wenigstens ein Geliermittel.

**Revendications**

**1.** Composition pharmaceutique transdermique pour une administration transdermique dans une surface cutanée, dans laquelle ladite composition comprend :

(i) 0,01 à 10 % (en poids/poids), de préférence 2 à 5 % (en poids/poids) d'endoxifène ou d'un sel pharmaceutiquement acceptable de celui-ci,
(ii) 60 à 80 % (en poids/poids) d'au moins un monoalcool, dans lequel le monoalcool est choisi parmi des monoalcools en $C_2$-$C_4$, de préférence de l'éthanol ou de l'isopropanol,
(iii) 0,01 à 10 % (en poids/poids) d'au moins un agent améliorant la pénétration, dans lequel le au moins un agent améliorant la pénétration est un acide oléique, à condition que le myristate d'isopropyle soit exclu,
(iv) 0 à 5 % (en poids/poids) d'au moins un agent gélifiant, de préférence, des acides polyacryliques, des cellulosiques ou des mélanges de ceux-ci, et
(v) quantité suffisante d'un véhicule aqueux jusqu'à 100 % (en poids/poids).

**2.** Composition pharmaceutique transdermique selon la revendication 1, dans laquelle le au moins un agent améliorant la pénétration est un mélange d'acide oléique et de propylène glycol.

**3.** Composition pharmaceutique transdermique selon la revendication 1, destinée à être utilisée dans une méthode d'administration d'une quantité thérapeutiquement efficace d'endoxifène ou de son sel pharmaceutiquement acceptable, à un patient en ayant besoin, comprenant l'administration transdermique de ladite composition dans une surface de peau dudit patient.

**4.** Composition pharmaceutique transdermique à utiliser selon la revendication 3, dans laquelle le patient souffre d'un trouble mammaire.

**5.** Composition pharmaceutique transdermique à utiliser selon la revendication 3, dans laquelle le patient souffre d'un déséquilibre en œstrogènes.

**6.** Composition pharmaceutique transdermique à utiliser selon la revendication 5, dans laquelle le déséquilibre en œstrogènes est un trouble mammaire choisi parmi des maladies mammaires bénignes, la gynécomastie, le cancer du sein, la mastalgie et des conditions impliquant un tissu mammaire dense

**7.** Paquet de dose, paquet de dose unitaire ou un paquet de doses multiples contenant une composition pharmaceutique transdermique selon la revendication 1.

**8.** Distributeur, de manière facultative avec pompe à main, contenant une composition pharmaceutique transdermique selon la revendication 1.

**9.** Procédé de préparation d'une composition pharmaceutique transdermique selon la revendication 1 comprenant les étapes de préparation d'un mélange comprenant :

(i) au moins un monoalcool choisi parmi des monoalcools en $C_2$-$C_4$,
(ii) de l'endoxifène ou des sels pharmaceutiquement acceptables de celui-ci,
(iii) un véhicule aqueux,
(iv) au moins un agent améliorant la pénétration, dans lequel le au moins un agent améliorant la pénétration est un acide oléique, à condition que le myristate d'isopropyle soit exclu, et
(v) de manière facultative, au moins un agent gélifiant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008070463 A
- WO 9527569 A
- WO 9615045 A
- US 5630531 A

**Non-patent literature cited in the description**

- **POTTS ; GUY.** *Pharm Res.,* 1992, vol. 9 (5), 663-9
- **CLEEK ; BUNGE.** *Pharm Res,* 1993, vol. 10 (4), 497-506
- **BALAKIN et al.** *Current Medicinal Chemistry,* 2006, vol. 13, 223-241